# EUROPEAN PATENT SPECIFICATION

(11) **EP 2 017 335 B1**
(45) Date of publication and mention of the grant of the patent: **01.12.2010**
(21) Application number: 08166771.9
(22) Date of filing: 04.10.2002
(51) Int. Cl.: C12N 9/12, C12N 15/00, C12N 5/00, G01N 33/50

(54) **Mutated Abl kinase domains**
Mutante der kinase Domäne des ABL
Mutants de domaines de la kinase de l'ABL.

(30) Priority: 05.10.2001 US 327389 P; 12.10.2001 US 328740 P; 11.01.2002 US 347351 P
(43) Date of publication of application: 21.01.2009
(62) Divisional of application: 02782829.2
(73) Proprietor: Novartis AG, 4002 Basel (CH); Université Victor Segalen Bordeaux 2, 33076 Bordeaux Cedex (FR); Oregon Health and Science University, Portland, OR 97201-4753 (US); Technische Universität München, 80333 München (DE); University of Heidelberg, 69117 Heidelberg (DE); Centre Hospitalier Regional Universitaire de Lille, 59037 Lille Cédex (FR); MEDVET SCIENCE PTY. LTD., Stepney, SA 5069 (AU)
(72) Inventor: Barthe, Christophe, 33127, Martignas Sur Jalles (FR); Branford, Susan, Flagstaff Hill, South Australia 5159 (AU); Corbin, Amie, Portland, OR 97219 (US); Druker, Brian Jay, Portland, OR 97239 (US); Duyster, Justus, 81739, Munich (DE); Hochhaus, Andreas, 68163 Mannheim (DE); Hughes, Timothy, Kensington Gardens, South Australia 5068 (AU); Kreil, Sebastian, 68165, Mannheim (DE); Leguay, Thibaut, 33000, Bordeaux (FR); Mahon, Francois-Xavier, 33000 Bordeaux (FR); Marit, Gerald, 33200, Bordeaux (FR); Mueller, Martin, 69120, Heidelberg (DE); Peschel, Christian, 81739, Munich (DE); Preudhomme, Claude, 62150, Houdain (FR); Roche Lestienne, Catherine, 59800, Lille (FR); Rudzki, Zbigniew, Rostrevor, South Australia 5073 (AU)
(74) Representative: Gruber, Markus

(56) References cited:
- WO-A-02/102976
- VON BUBNOFF N ET AL: "BCR-ABL gene mutations in relation to clinical resistance of Philadelphia-chromosome-positive leukaemia to STI571: a prospective study" LANCET THE, LANCET LIMITED. LONDON, GB, vol. 359, no. 9305, 9 February 2002 (2002-02-09), pages 487-491, XP004790373 ISSN: 0140-6736
- BRANFORD SUSAN ET AL: "High frequency of point mutations clustered within the adenosine triphosphate-binding region of BCR/ABL in patients with chronic myeloid leukemia or Ph-positive acute lymphoblastic leukemia who develop imatinib (STI571) resistance" BLOOD, AMERICAN SOCIETY OF HEMATOLOGY, US, vol. 99, no. 9, 1 May 2002 (2002-05-01), pages 3472-3475, XP002420122 ISSN: 0006-4971
- ROUMIANTSEV SERGEI ET AL: "Clinical resistance to the kinase inhibitor STI-571 in chronic myeloid leukemia by mutation of Tyr-253 in the Abl kinase domain P-loop" PROCEEDINGS OF THE NATIONAL ACADEMY OF SCIENCES OF THE UNITED STATES OF AMERICA, vol. 99, no. 16, 6 August 2002 (2002-08-06), pages 10700-10705, XP002505152 ISSN: 0027-8424
- GORRE MERCEDES E ET AL: "Clinical resistance to STI-571 cancer therapy caused by BCR-ABL gene mutation or amplification" 20010803, vol. 293, no. 5531, 3 August 2001 (2001-08-03), pages 876-880, XP002229884

## Description

### Field of the Invention:

This invention relates to isolated polypeptides which comprise a functional kinase domain comprising the amino acid sequence of the native human Abl kinase domain or an essentially similar sequence thereof in which amino acid Gly250 is replaced by another amino acid, said mutated functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof, to the use of such polypeptides to screen for compounds which inhibit the tyrosine kinase activity of such polypeptides, to nucleic acid molecules encoding such polypeptides, to recombinant vectors and host cells comprising such nucleic acid molecules and to the use of such nucleic acid molecules in the production of such polypeptides for use in screening for compounds which inhibit the tyrosine kinase activity of such polypeptides.

### Background of the Invention:

Bcr-Abl, a constitutively activated tyrosine kinase resulting from the formation of the Philadelphia chromosome [Nowell P.C. and Hungerford D.A., Science 132, 1497 (1960)] by reciprocal translocation between the long arms of chromosomes 9 and 22 [Rowley J.D., Nature 243, 290-293 (1973)], has been established as the characteristic molecular abnormality present in virtually all cases of chronic myeloid leukemia (CML) and up to 20 percent of adult acute lymphoblastic leukemia (ALL) [Faderl S. et al., N Engl J Med 341, 164-172 (1999); Sawyers C.L., N Engl J Med 340, 1330-1340 (1999)]. Bcr-Abl is sufficient to cause CML in mice [Daley G.Q. et al., Science 247, 824-830 (1990)] and its transforming capacity is absolutely dependent on tyrosine kinase activity [Lugo T.G. et al., Science 247, 1079 (1990)]. The compound N-[4-methyl-3-(4-pyridin-3-yl-pyrimidin-2-ylamino)-phenyl]-4-(4-methyl-piperazin-1-ylmethyl)-benzamide (hereinafter also referred to as "STI571"; STI571 is described in EP 0 564 409 and, in the form of the methane sulfonate salt, in WO 99/03854), a competitive inhibitor at the ATP-binding site of Bcr-Abl, as well as of the receptor for platelet-derived growth factor, and c-kit tyrosine kinase [Lugo T.G. et al., Science 247, 1079 (1990)], has been shown to be capable of very rapidly reversing the clinical and hematological abnormalities of CML in chronic phase and in blast crisis as well as of Ph chromosome-positive (Ph+) acute lymphoblastic leukemia (Ph+ ALL) [Druker B.J. et al., N Engl J Med 344, 1031-1037 (2001); Druker B.J. et al., N Engl J Med 344, 1038-1042 (2001)]. Whereas almost all chronic phase CML patients durably respond, remissions in CML blast crisis and Ph+ ALL are transient, and most patients relapse after several months, despite continued therapy with STI571 [Druker B. J. et al., N Engl J Med 344, 1038-1042 (2001)]. The mechanism of resistance to STI571 is subject of intense research.

It was now surprisingly found that mutations present in the kinase domain of the Bcr-Abl gene of patients suffering from CML or Ph+ ALL account for the biological resistance of these patients towards STI571 treatment in that said mutations lead to resistance of the Bcr-Abl tyrosine kinase towards inhibition by STI571. For instance, Gorre M.E. et al.. Science, 293, 876-880 (2001) report that a Thr→Ile substitution in the Abl kinase domain can confer STE571 resistance.

These findings are extremely valuable in e.g. finding new compounds or combinations of compounds which are capable to overcome resistance towards treatment with STI571. Moreover, knowledge of such mutations is also very useful in the diagnosis of Ph+ leukemias in that it allows e.g. the detection of drug-resistant clones before clinical relapse of the patient.

### Definitions:

Within the context of this disclosure the following expressions, terms and abbreviations have the meanings as defined below:
In the expression "a functional kinase domain", the term "functional" indicates that the respective kinase domain possesses tyrosine kinase activity. Preferably, the kinase activity of such a functional kinase domain is in the range of that of the native human Abl kinase domain.
In the expression "a functional kinase domain being resistant to inhibition of its tyrosine kinase activity by STI571 or a salt thereof", the term "resistant" means that STI571 inhibits the respective functional kinase domain with an IC₅₀ that is higher than that of the native human Abl kinase domain, i.e. higher than about 0.025 µM, preferably higher than about 0.15 µM, more preferably higher than about 0.25 µM, most preferably higher than about 5 µM.
In the expression "amino acid sequence of the native human Abl kinase domain or an essentially similar sequence thereof", the part "or an essentially similar sequence thereof" refers to the amino acid sequence of the native human Abl kinase domain containing mutations, including amino acid exchanges, amino acid deletions and/or amino acid additions, that are not essential for the functionality of the kinase and its resistance to inhibition by STI571 or a salt thereof within the meaning of the term "functional" and "resistant" as defined hereinabove.
The expression "replaced by another amino acid" refers to the replacement of a certain natural amino acid by another natural amino acid.
The names of the amino acids are either written out or the one letter or three letter codes are used. Mutations are referred to by accepted nomenclature, e.g. "Ala380Thr" or "380 Ala→Thr" both indicating that alanine at position 380 is replaced by threonine.
SEQ ID NO:1 represents the cDNA coding for the native human Abl protein (human c-abl mRNA; GenBank Accession No.: X16416).
SEQ ID NO:2 represents the amino acid sequence of the native human Abl protein (human c-Abl; SwissProt Acc. No.: P00519).
Unless indicated otherwise, the number given for a certain amino acid refers to the numbering of the amino acids in SEQ ID NO:2. In an amino acid sequence that is essentially similar to the amino acid sequence of the native human Abl kinase domain within the meaning as defined above, the amino acids are numbered in accordance with the numbering of the amino acids in SEQ ID NO:2.
The term "isolated" means that the material is removed from its original environment (e.g., the natural environment if it is naturally occurring).
A "host cell", refers to a prokaryotic or eukaryotic cell that contains heterologous DNA that has been introduced into the cell by any means, e.g., electroporation, calcium phosphate precipitation, microinjection, transformation, viral infection, and the like.

### Description of the Invention:

In practicing the present invention, many conventional techniques in molecular biology, microbiology, and recombinant DNA are used. These techniques are well known and are explained in, for example, Current Protocols in Molecular Biology, Volumes I, II, and III, 1997 (F. M. Ausubel ed.); Sambrook et al., 1989, Molecular Cloning: A Laboratory Manual, Second Edition, Cold Spring Harbor Laboratory Press, Cold Spring Harbor, N.Y.; DNA Cloning: A Practical Approach, Volumes I and II, 1985 (D. N. Glover ed.); Oligonucleotide Synthesis, 1984 (M. L. Gait ed.); Nucleic Acid Hybridization, 1985, (Hames and Higgins); Transcription and Translation, 1984 (Hames and Higgins eds.); Animal Cell Culture, 1986 (R. I. Freshney ed.); Immobilized Cells and Enzymes, 1986 (IRL Press); Perbal, 1984, A Practical Guide to Molecular Cloning; the series, Methods in Enzymology (Academic Press, Inc.); Gene Transfer Vectors for Mammalian Cells, 1987 (J. H. Miller and M. P. Calos eds., Cold Spring Harbor Laboratory); and Methods in Enzymology Vol. 154 and Vol. 155 (Wu and Grossman, and Wu, eds., respectively).

In particular, the polypeptides of the present invention can be produced by recombinant DNA technology using techniques well-known in the art. Methods which are well known to those skilled in the art can be used to construct expression vectors containing the sequences encoding the polypeptides of the invention and appropriate transcriptional/translational control signals. A variety of host-expression vector systems can be utilized to express the polypeptides of the invention.
(1) The invention relates to an isolated polypeptide which comprises a functional kinase domain comprising the amino acid sequence of the native human Abl kinase domain or an essentially similar sequence thereof in which at least Gly250 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(2) Similarly, the invention relates especially to an isolated polypeptide which comprises a functional kinase domain comprising the amino acid sequence of the native human Abl kinase domain or an essentially similar sequence thereof that contains the amino acid mutation Gly250Glu, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(3) Similarly, the invention relates especially to an isolated polypeptide which comprises a functional kinase domain comprising the amino acid sequence of the native human Abl kinase domain or an essentially similar sequence thereof that contains the amino acid mutation Gly250Ala, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.
(4) In a preferred embodiment the invention relates to an isolated polypeptide according to any one of the preceding paragraphs (1) - (3), wherein the amino acid sequence of the native human Abl kinase domain consists of amino acids 229-500 of SEQ ID NO:2.
(5) In another preferred embodiment the invention relates to an isolated polypeptide according to any one of the preceding paragraphs (1) - (4), said isolated polypeptide being a Bcr-Abl tyrosine kinase.
(6) In yet another preferred embodiment the invention relates to the use of an isolated polypeptide of any one of the preceding paragraphs (1) - (5) to screen for compounds which inhibit the tyrosine kinase activity of said polypeptide.
(7) The invention also relates to an isolated nucleic acid molecule comprising a nucleotide sequence that encodes a polypeptide according to any one of the preceding paragraphs (1) - (5).
(8) The invention further relates to the use of a nucleic acid molecule of the preceding paragraph (53) in the production of a polypeptide of any one of the preceding paragraphs (1) - (5) for use in screening for compounds which inhibit the tyrosine kinase activity of said polypeptide.
(9) The invention also relates to a recombinant vector comprising a nucleic acid molecule according to the preceding paragraph (7).
(10) The invention further relates especially to a recombinant vector according to the preceding paragraph (9), which is a recombinant expression vector.
(11) The invention also relates to a host cell comprising a recombinant vector according to the preceding paragraph (9) or (10).

Preferably the invention relates to an isolated polypeptide which comprises a functional kinase domain comprising the amino acid sequence of the native human Abl kinase domain in which at least one amino acid is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methyl-piperazin-1-ylmethyl)-benzamide or a salt thereof.

A preferred salt of STI571 is the methane sulfonate salt described in WO 99/03854.

Screening for compounds which inhibit the tyrosine kinase activity of the polypeptides of the invention may be done for example by using an isolated polypeptide of the invention in any *in vitro* tyrosine kinase phosphorylation assay known in the art and determining the potential of a compound to inhibit the tyrosine kinase activity of a polypeptide of the invention in such an assay.

High-throughput screening assays known in the art may be used to screen large compound libraries for compounds which inhibit the tyrosine kinase activity of the polypeptides of the invention.

Besides the random screening of large compound libraries, the polypeptides of the present invention may also be used in the following screening approach: The 3-dimensional structure of a polypeptide of the invention is determined by e.g. X-ray crystallography. The atomic coordinates of a polypeptide of the invention are then used to design a potential inhibitor. Said potential inhibitor is then synthesized and tested for its ability to inhibit the tyrosine kinase activity of the polypeptide of the invention in any *in vitro* tyrosine kinase phosphorylation assay.

### Exampes:

The following Examples serve to illustrate the invention without limiting its scope. Mutations are amino acids other than residue 250 are disclosed for reference.

### Example 1:

A PCR strategy was used to amplify the ATP binding region of Bcr-Abl cDNA and the entire region was sequenced in both directions. Bcr-Abl mutations were found in 4/6 patients on STI571 for blast crisis CML (2 myeloid, 2 lymphoid BC), accelerated phase (1) or second chronic phase CML (1) who had developed haematological resistance to STI571. One patient had the mutation Thr315Ile. Two patients had mutations at position 250, which substituted glycine for glutamic acid. One patient had a mutation at amino acid 253, which substituted tyrosine for histidine. Amino acid 250 does not form a hydrogen bond with STI571, as does amino acid 315, nor is it involved in van der Waals interaction with the inhibitor, as is amino acid 253. Where samples were available (3 cases) we confirmed that the mutation was not present prior to STI571 therapy, nor was it present in the normal Abl allele. We also sequenced the ATP binding region of Bcr-Abl in 8 patients with advanced phase (5) or chronic phase (3) CML who had achieved and maintained haematological responses but only had minor or no cytogenetic response on STI571. None of these patients had evidence of mutations after 6-9 months of STI571 therapy. We postulate that several point mutations in Bcr-Abl emerge in patients with advanced phase CML which are likely to totally or partially abrogate STI571 binding to Bcr-Abl.

### Example 2:

An RT-PCR strategy was used to amplify and sequence the Abl kinase domain of Bcr-Abl in 28 patients. We selected STI571-refractory and STI571-resistant patients from 253 patients enrolled in expanded access studies at 5 Australian centres. The aim was to determine the frequency and timing of acquired mutations within carefully defined clinical groups, determine their distribution within the Bcr-Abl kinase domain and identify any association between specific mutations and clinical features.

### Study Design

STI571-resistant patients (n=18) were defined as those with a loss of complete haematological remission which had been present for at least 3 months or evolution to acute phase CML or relapsed Ph+ ALL. STI571-refractory patients (n=10) were those who failed to achieve a major cytogenetic response after at least 6 months of therapy.

Extraction of RNA from blood, reverse transcription and DNA sequencing procedures have been previously described [Branford S. et al., Br. J. Haematol. 107, 587-599 (1999)]. A long PCR method [Branford S. et al., Br. J. Haematol. 109, 635-637 (2000)] was used to amplify the Abl kinase domain of Bcr-Abl with forward primer BcrF (5' tgaccaactcgtgtgtgaaactc) and reverse primer AblKinaseR (5' tccacttcgtctgagatactggatt). A second stage PCR used forward primer AblkinaseF (5' cgcaacaagcccactgtct) and reverse primer AblkinaseR. The entire kinase domain was sequenced, an area including 863 bases (GenBank accession number M14752).

### Results

### STI571-resistant patients:

Twelve of 18 STI571-resistant patients had mutations in the ATP binding region of Bcr-Abl (Table 6). In 9 cases where samples were available, we confirmed that the mutation was not present before starting STI571 therapy, nor was it present in 4 cases tested at 3-9 months, which in each case was before the onset of resistance. Three of the 6 resistant patients without Bcr-Abl mutations had evidence of clonal evolution at the time of relapse including an additional Philadelphia (Ph) chromosome in 2 cases. One patient had both a mutation and an extra Ph chromosome at time of relapse.

The 6 mutations identified are T315I (n=3 patients), Y253H (n=1), F317L (n=1), E255K (n=4), G250E (n=2) and M351T (n=1).

The 18 STI571-resistant patients could be subdivided into those who relapsed into blast crisis or Ph+ ALL (n=8), those relapsing into accelerated phase (n=6) and those with evidence of loss of hematological remission who remained in chronic phase (n=4). All 3 groups included patients with mutations. Six of the 8 patients who relapsed directly into blast crisis/ALL had mutations. In 3 of these the T315I mutation was present. Two of the 6 patients relapsing into accelerated phase had mutations. The remaining 4 patients relapsing into chronic phase all had mutations.

### STI571-refractory patients:

Only 1 of 10 refractory patients had a mutation (Table 6). There was no evidence of the mutation pre-STI571 therapy or 3 months after starting treatment. The mutant clone mixed with wild-type Bcr-Abl emerged at 8 months and persisted in a mixed pattern until the mutant clone became predominant at 11 months. To date there has been no clinical evidence of resistance in this patient.

**Table 6. Clinical course and mutation analysis of patients treated with STI571**

| Patient ID | Age /Sex | Disease status at start of STI571* | Duration of STI571 Treatment | Best Response to STI571* | Response at Time of Mutation Analysis* | Time of Mutation Analysis † | Mutation Result | Nucleotide Substitution (GenBank no. M14752) |
|---|---|---|---|---|---|---|---|---|
| Emerging Resistance | | | | | | | | |
| 01 | 61 F | 3 (3^{rd}) | 9m | | 1b | Pre Study | NM | |
| | | | | 7 | 7 | 3m | NM | |
| | | | | | 7 | 6m | NM | |
| | | | | | 1b | 9m | T315I | G to C nt 944 |
| 02 | 75 F | 1a | 4.5m | | 1a | Pre study | | |
| | | | | 4 | 1a | 4.5m | T315I | G to C nt 944 |
| 03 | 62 M | 1b | 2m | | 1b | Pre study | | |
| | | | | 4 | 1b | 2m | T315I | G to C nt 944 |
| 04§ | 59 F | 1c | 3m | 7 | 1c | Pre Study | NM | |
| | | | | | 1c | 5m | Y253H | T to C nt 757 |
| | | | | | 1c | 5.5m | Y253H | T to C nt 757 |
| 05 | 40 M | 3 | 8m | | 3 | Pre Study | NM | |
| | | | | 5 | 5 | 4m | F317L | C to G nt 951 |
| | | | | | 4 | 7m | F317L | C to G nt 951 |
| 06 | 66 M | 1b | 8m‡ | 4 | 1b | 8m | G250E | G to C nt 749 |
| 07 | 54 F | 1a | 10.5m | | 1a | Pre Study | NM | |
| | | | | 5 | 5 | 6m | NM | |
| | | | | | 5 | 9m | NM | |
| | | | | | 5 | 10m | G250E | G to C nt 749 |
| | | | | | 4 | 10.5m | G250E | G to C nt 749 |
| 08 | 41 M | 2 | 6m | 5 | 2 | 6m | E255K | G to A nt 769 |
| 09 | 60 M | 2 | 5m | 5 | 5 | 3m | NM | |
| | | | | | 4 | 6m | E255K | G to A nt 769 |
| 10 | 44 M | 2 | 4.5m | | 2 | Pre Study | NM | |
| | | | | 5 | 4 | 4m | E255K | G to A nt 769 |
| 11§ | 60 F | 1c | 3m | | 1c | Pre Study | NM | |
| | | | | 5 | 1c | 3m | E255K | G to A nt 769 |
| 12 | 52 F | 3 | 9m | | 3 | Pre Study | NM | |
| | | | | 7 | 7 | 6m | NM | |
| | | | | | 7 | 7m | NM | |
| | | | | | 6 | 8m | M351T | T to C nt 1052 |
| | | | | | 2 ^{P} | 9m | M351T | T to C nt 1052 |
| 13 | 64 M | 3 | 10.5m | 5 | 2 ^{P} | 10m | NM | |
| 14 | 78 F | 1a | 8m | 5 | 2 ^{C+P} | 8m | NM | |
| 15 | 47 M | 2^{C} | 9m | 2 ^{C} | 1 ^{C} | 7m | NM | |
| 16 | 56 F | 3 | 7m | 5 | 2 | 6m | NM | |
| 17 | 37 M | 1a | 7m | 4 | 1a | 4.5m | NM | |
| 18 | 63 M | 2 | 7m | 4 | 2 | 8m | NM | |
| | | | | | | | | |

| Refractory | | | | | | | | |
|---|---|---|---|---|---|---|---|---|
| 19 | 55 F | 2 ^{C} | 6m | 4 | 4 ^{C} | 3.5m | NM | |
| 20 | 63 M | 2 ^{C} | 11m | | 2 ^{C} | Pre Study | NM | |
| | | | | 2 ^{C} | 2 ^{C} | 3m | NM | |
| | | | | | 2 ^{C} | 8m | M351T | T to C nt 1052 |
| | | | | | 2 ^{C} | 9m | M351T | T to C nt 1052 |
| | | | | | 2 ^{C} | 10m | M351T | T to C nt 1052 |
| | | | | | 2 ^{C} | 11m | M351T | T to C nt 1052 |
| 21 | 62 F | 2 | 10.5m | 5 | 4 | 9m | NM | |
| 22 | 54 M | 2 | 8m | 5 | 5 | 6m | NM | |
| 23 | 61 F | 3 | 6m | 5 | 4 | 5m | NM | |
| 24 | 61 F | 3 | 10.5m | 5 | 4 | 6m | NM | |
| 25 | 47 F | 3 | 10m | 5 | 5 | 6m | NM | |
| 26 | 40 F | 3 | 6m | 5 | 5 | 3m | NM | |
| 27 | 60 M | 3 | 6m | 5 | 5 | 4m | NM | |
| 28 | 42 M | 3 | 7m | 5 | 5 | 6m | NM | |

| | | | | | | | | |
|---|---|---|---|---|---|---|---|---|
| F: female; M: male; m: months. NM Indicates no mutation detected following sequencing of the kinase region of Bcr-Abl in both directions. ^{C}Indicates clonal evolution. ^{P}Indicates double Philadelphia chromosome. *Disease Status/Response: 1 a) myeloid blast crisis b) lymphoid blast crisis c) Ph-positive ALL relapse. 2) Accelerated Phase, 3) Chronic Phase, 4) Partial Response (blasts in blood + bone marrow < 15%), 5) Complete Haematologic Response (WBC<10.0, Plts < 450, no blasts, myelocytes + metamyelocytes < 5%, no promyelocytes, no disease-related symptoms or extramedullary disease), 6) Major Cytogenetic Response, 7) Complete Cytogenetic Response. †Months since treatment start. ‡Although patient was on study for 8 months, STI571 was alternated monthly with oral arsenic and therefore the patient received only 4 months of STI571 over this period. §Ph-positive ALL patients. | | | | | | | | |

Further studies revealed the presence of two additional mutations, i.e. Met244Val and Phe486Ser, in STI571-resistant/refractory patients suffering from Ph chromosome-positive leukemia.

In another study, CML patients from 12 centers within Australia and New Zealand were tested for mutation analysis. The samples were primarily collected for molecular assessment of BCR-ABL levels and only proceeded to mutation analysis if stored RNA contained a measurable level of BCR-ABL and the control gene level indicated non-degraded RNA. Patients had either received 6 or more months of imatinib therapy or had developed resistance and ceased therapy before 6 months (n=2). Samples from 156 patients were available but 12 could not be tested, 4 due to low levels of BCR-ABL and 8 due to inadequate RNA quality. The remaining 144 patients were grouped according to the disease stage at start of imatinib; 40 in AP, 64 in late-CP defined as ≥ 12 months since diagnosis and 40 in early-CP defined as <12 months since diagnosis. Sixteen of the early-CP patients had failed previous interferon therapy and 24 had only received hydroxyurea prior to imatinib therapy. Response to imatinib was categorised by the cytogenetic analysis at 6 months as either a major cytogenetic response (MCR) if the number of Philadelphia chromosome positive cells was <35% or no MCR if 35-100%. Imatinib resistance was defined as loss of a complete hematologic remission (CHR) that had been present for at least 3 months, loss of CHR with transformation to accelerated or blastic phase, or loss of an established MCR or a complete cytogenetic response (CCR defined as Philadelphia chromosome negative). 346 RNA samples were analysed as already mentioned herein. Depending on available RNA, patients were tested for mutations at between 1-15 different time-points. The median duration of imatinib therapy was therefore determined from the last time-point of analysis. AP patients had received a median of 9 months of imatinib (range 4 to 24), late-CP 10 months (range 6-24) and early-CP 14 months (range 5-24).

### Imatinib resistance in accelerated phase patients

Fourteen of 40 AP patients developed imatinib resistance, 5 with transformation to blast phase, 8 with recurrence of AP and 1 with loss of CCR. Mutations were detected in 12 of 14 (86%) resistant patients at a median of 8 months of imatinib therapy (range 4-13).

### Imatinib resistance in late chronic phase patients (late-CP)

Thirteen of 64 late-CP patients developed imatinib resistance, 5 with transformation to blast phase, 6 to AP and 2 lost a MCR. Mutations were detected in 11 of 13 (85%) resistant patients at a median of 8 months of imatinib therapy (range 3-18).

### Imatinib resistance in early chronic phase patients (early-CP)

Six of 40 early-CP patients developed resistance, 1 transformed to blast crisis, 2 to AP, 2 lost CCR and 1 lost MCR. No mutations were detected in the resistant patients.

### Duration of CML and the development of mutations

When all the patients in the study who achieved a major cytogenetic response (MCR) within 6 months were studied, those treated >4 years since diagnosis had a 9 times higher incidence of mutations than those treated within 4 years (6 of 22 (27%) versus 2 of 72 (3%)). When all the patients who did not achieve a MCR within 6 months were studied, those treated >4 years since diagnosis had a 2 times higher incidence of mutations than those treated within 4 years (12 of 22 (54%) versus 7 of 28 (25%)). The highest incidence of mutations was in AP patients treated >4 years from diagnosis and who failed to achieve a MCR by 6 months (8 of 12, 67%). In this study 27 of the 144 patients developed mutations. The median duration of CML prior to commencing imatinib therapy of the 27 patients with mutations (5.3 years, range 1.1 to 11) was statistically different to the 117 patients without mutations (1.3 years, range 0.02-17.7) p<0.0001.

### Mutations in the BCR-ABL kinase domain

Table 7 details the 17 different mutations in the BCR-ABL kinase domain detected in 27 patients. These were all point mutations and were located within a sequence of 728 nucleotides involving amino acids 244 to 486. Seven patients had 2-4 mutations and one patient had 2 different mutations at the same nucleotide which both altered the amino acid at position 252 from glutamine to histidine. The mutations, L248V at the N-terminal and S417Y, E459K and F486S at the C-terminal of the kinase domain have not previously been described. The first 3 mutations were all detected in one imatinib resistant patient who also had the G250E mutation.

**Table 7. BCR-ABL kinase domain mutations**

| Mutation | Nucleotide Substitution (GenBank number M14752) | Number of patients with the mutation* |
|---|---|---|
| M244V | 730A>G | 1 |
| L248V | 742C>G | 1 |
| G250E | 749G>A | 3 |
| Q252H | 756G>C | 3 |
| Q252H | 756G>T | 1 |
| Y253F | 758A>T | 2 |
| E255K | 763G>A | 1 |
| E255V | 764A>T | 5 |
| T315I | 944C>T | 2 |
| F317L | 951C>G | 2 |
| M351T | 1052T>C | 8 |
| E355G | 1064T>C | 3 |
| F359V | 1075T>G | 2 |
| H396R | 1187A>G | 1 |
| S417Y | 1250C>A | 1 |
| E459K | 1375G>A | 1 |
| F486S | 1457T>C | 1 |

| | | |
|---|---|---|
| *7 patients had multiple mutations | | |

### Example 3:

In this example we studied 43 CML patients (18 in chronic phase and 25 in advanced phase of the disease) who become either cytogenetical or hematological resistant to imatinib mesylate treatment. The advanced phase patients were treated with imatinib mesylate during a median time of 3 months and chronic phase patients during a median time of 13 months. After a first amplification including *BCR-ABL* fusion (1300pb), we carried out nested PCR to amplify a fragment of 584pb including the ATP binding domain and a fragment of 386pb containing the SH2 and SH3 domains. PCR products were sequenced allowing to cover the different ABL domains including amino acid 72 to 180 and amino acid 234 to 396 to study respectively SH2/SH3 and ATP binding domains.

Regarding the ATP binding domain among the 43 patients, 5 cases of mutations were detected. Three patients (one in accelerated phase, and 2 in blast crisis) exhibited the T315I mutation. In one other accelerated phase patient the E255K mutation was detected. In a cytogenetic resistant patient (in chronic phase) treated by imatinib mesylatemore than one year, the investigations found a newly described Gly250Ala substitution. Regarding the SH2 and SH3 domains no mutation were detected in the different samples providing from the all the 43 CML patients studied.

Our data confirm that in CML patients treated with STI571, ABL mutations are not restricted to the accelerated phase of the disease and we report a new point mutation not described before.

After in vitro mutagenesis, Ba/F3 cell lines were engineered to express either wild-type and T315I, E255K, A250G mutants, in the aim to study the differential sensitivity to imatinib mesylate. Preliminary results confirmed the high level of resistance from the BaF/BCR-ABL*T315I. The other functional studies are in progress in the laboratory and be will compared to this one.

## Claims

1. An isolated polypeptide which comprises a functional kinase domain comprising the amino acid sequence of the native human Abl kinase domain or an essentially similar sequence thereof in which Glycine 250 is replaced by another amino acid, said functional kinase domain being resistant to inhibition of its tyrosine kinase activity by N-[4-methyl-3-(4-pyridin-3-yl-pyrimidin-2-ylamino)-phenyl]-4-(4-methylpiperazin-1-ylmethyl)-benzamide or a salt thereof.

2. The isolated polypeptide according to claim 1, wherein Glycine 250 is replaced by glutamic acid.

3. The isolated polypeptide according to any one of claims 1 to 2, wherein the amino acid sequence of the native human Abl kinase domain consists of amino acids 229-500 of SEQ ID NO: 2.

4. An isolated polypeptide according to any one of claims 1 to 3, which is a Bcr-Abl tyrosine kinase.

5. Use of a polypeptide according to any one of claims 1 to 4 to screen for compounds which inhibit the tyrosine kinase activity of said polypeptide.

6. An isolated nucleic acid molecule comprising a nucleotide sequence that encodes a polypeptide according to any one of claims 1 to 4.

7. Use of a nucleic acid molecule according to claim 6 in the production of a polypeptide according to any one of claims 1 to 4 for use in screening for compounds which inhibit the tyrosine kinase activity of said polypeptide

8. A recombinant vector comprising a nucleic acid molecule according to claim 6.

9. A recombinant vector according to claim 8, which is a recombinant expression vector.

10. A host cell comprising a recombinant vector according to claim 8 or 9.

11. A method of identifying G250E mutation of Bcr-Abl in patients refractory or resistant to the treatment of N-[4-methyl-3-(4-pyridin-3-yl-pyrimidin-2-ylamino)-phenyl]-4-(4-methyl-piperazin-1-ylmethyl)-benzamide or a salt thereof, the method comprising determining the nucleotide sequence of the ATP binding region of Bcr-Abl cDNA, and determining whether Glycine 250 is replaced by glutamic acid.

## Patentansprüche

1. Isoliertes Polypeptid, das eine funktionale Kinasedomäne, die die Aminosäuresequenz der nativen humanen Abl-Kinasedomäne oder eine im Wesentlichen ähnliche Sequenz hiervon umfasst, worin Glycin 250 durch eine andere Aminosäure ersetzt ist, umfasst, wobei die funktionale Kinasedomäne gegenüber einer Hemmung ihrer Tyrosinkinaseaktivität durch N-[4-Methyl-3-(4-pyridin-3-yl-pyrimidin-2-ylamino)-phenyl]-4-(4-methylpiperazin-1-ylmethyl)-benzamid oder ein Salz hiervon resistent ist.

2. Isoliertes Polypeptid nach Anspruch 1, wobei Glycin 250 durch Glutaminsäure ersetzt ist.

3. Isoliertes Polypeptid nach einem der Ansprüche 1 bis 2, wobei die Aminosäuresequenz der nativen humanen Abl-Kinasedomäne aus den Aminosäuren 229-500 der SEQ ID Nr. 2 besteht.

4. Isoliertes Polypeptid nach einem der Ansprüche 1 bis 3, bei dem es sich um eine Bcr-Abl-Tyrosinkinase handelt.

5. Verwendung eines Polypeptids nach einem der Ansprüche 1 bis 4 zum Screenen bezüglich Verbindungen, die die Tyrosinkinaseaktivität des Polypeptids hemmen.

6. Isoliertes Nukleinsäuremolekül, das eine Nucleotidsequenz umfasst, die für ein Polypeptid nach einem der Ansprüche 1 bis 4 kodiert.

7. Verwendung eines Nukleinsäuremoleküls nach Anspruch 6 bei der Herstellung eines Polypeptids nach einem der Ansprüche 1 bis 4 zur Verwendung beim Screenen bezüglich Verbindungen, die die Tyrosinkinaseaktivität des Polypeptids hemmen.

8. Rekombinanter Vektor, der ein Nukleinsäuremolekül nach Anspruch 6 umfasst.

9. Rekombinanter Vektor nach Anspruch 8, bei dem es sich um einen rekombinanten Expressionsvektor handelt.

10. Wirtszelle, die einen rekombinanten Vektor nach Anspruch 8 oder 9 umfasst.

11. Verfahren zur Identifizierung einer G250E-Mutation von Bcr-Abl bei Patienten, die gegenüber der Behandlung mit N-[4-Methyl-3-(4-pyridin-3-yl-pyrimidin-2-ylamino)-phenyl]-4-(4-methylpiperazin-1-ylmethyl)-benzamid oder einem Salz hiervon refraktär oder resistent sind, wobei das Verfahren ein Bestimmen der Nukleotidsequenz der ATP-Bindungsregion der Bcr-Abl-cDNA und ein Bestimmen, ob Glycin 250 durch Glutaminsäure ersetzt ist, umfasst.

## Revendications

1. Polypeptide isolé qui comprend un domaine fonctionnel de la kinase comprenant la séquence d'acides aminés du domaine de la kinase humaine native Abl ou une séquence essentiellement similaire de celui-ci dans laquelle la glycine 250 est remplacée par un autre acide aminé, ledit domaine fonctionnel de la kinase étant résistant à l'inhibition de son activité tyrosine kinase par le N-[4-méthyl-3-(4-pyridin-3-yl-pyrimidin-2-ylamino)-phényl]-4-(4-méthylpipérazin-1-ylméthyl)-benzamide ou un sel de celui-ci.

2. Polypeptide isolé selon la revendication 1, dans lequel la glycine 250 est remplacée par l'acide glutamique.

3. Polypeptide isolé selon l'une des revendications précédentes, dans lequel la séquence d'acides aminés du domaine de la kinase humaine native Abl se compose des acides aminés 229-500 de l'ID SEQ. n° 2.

4. Polypeptide isolé selon l'une quelconque des revendications 1 à 3, qui est une tyrosine kinase Bcr-Abl.

5. Utilisation d'un polypeptide selon l'une quelconque des revendications 1 à 4 pour le criblage de composés qui inhibent l'activité tyrosine kinase dudit polypeptide.

6. Molécule d'acide nucléique isolée comprenant une séquence nucléotidique qui code pour un polypeptide selon l'une quelconque des revendications 1 à 4.

7. Utilisation d'une molécule d'acide nucléique selon la revendication 6 dans la production d'un polypeptide selon l'une quelconque des revendications 1 à 4, à utiliser dans le criblage de composés qui inhibent l'activité tyrosine kinase dudit polypeptide.

8. Vecteur recombinant comprenant une molécule d'acide nucléique selon la revendication 6.

9. Vecteur recombinant selon la revendication 8, qui est un vecteur d'expression recombinant.

10. Cellule hôte comprenant un vecteur recombinant selon la revendication 8 ou 9.

11. Procédé d'identification de la mutation G250E de Bcr-Abl chez des patients réfractaires ou résistant au traitement du N-[4-méthyl-3-(4-pyridin-3-yl-pyrimidin-2-ylamino)-phényl]-4-(4-méthylpipérazin-1-ylméthyl)-benzamide ou d'un sel de celui-ci, le procédé comprenant l'analyse de la séquence nucléotidique de la région de liaison à l'ATP de l'ADNc Bcr-Abl, et la recherche pour savoir si la glycine 250 est remplacée par l'acide glutamique.
